# EUROPÄISCHE PATENTANMELDUNG

(11) **EP 4 252 710 A1**
(43) Veröffentlichungstag der Anmeldung: **04.10.2023**
(21) Anmeldenummer: 23164552.4
(22) Anmeldetag: 28.03.2023
(51) Int. Cl.: A61D 7/00, A61D 1/02

(54) **VORRICHTUNG ZUM NADELLOSEN INJIZIEREN EINES FLUIDS IN EIN TIER**

(30) Priorität: 29.03.2022 DE 102022107416
(71) Anmelder: Henke-Sass, Wolf GmbH, 78532 Tuttlingen (DE)
(72) Erfinder: ALTERMANN, Frank, 78532 Tuttlingen (DE); WIEDMANN, Maikel, 78194 Immendingen (DE); SAUTER, Robin, 78532 Tuttlingen (DE); SCHMIDT, Anika, 78567 Fridingen (DE)
(74) Vertreter: Patentanwälte Geyer, Fehners & Partner mbB

(57) **Zusammenfassung**

Es wird eine Vorrichtung zum nadellosen Injizieren eines Fluids in ein Tier bereitgestellt, wobei die Vorrichtung
eine Anlageeinrichtung (3), die eine geformte Anlagefläche (6) aufweist, die entsprechend einem Körperteil des Tieres geformt ist, dem die Injektion verabreicht werden soll,
einen ersten Injektor (10) zum nadellosen Injizieren, der ein vorderes Abgabeende (9) aufweist, das sich beim nadellosen Injizieren durch eine erste Durchgangsöffnung (7) in der geformten Anlagefläche (6) erstreckt und gegenüber der geformten Anlagefläche (6) in einem Bereich angrenzend an die erste Durchgangsöffnung (7) vorsteht,
eine erste Messeinrichtung (32, 45), die mindestens ein erstes Messsignal abgibt, um einen Kontakt des Tieres mit dem vorderen Abgabeende (9) des ersten Injektors (10) zu detektieren, und
eine Steuereinheit (S), die ein nadelloses Injizieren mittels des ersten Injektors (10) basierend auf dem mindestens einen ersten Messsignal aktiviert.

## Beschreibung

Die Erfindung betrifft eine Vorrichtung zum nadellosen Injizieren eines Fluids in ein Tier.

Bei der Haltung und Aufzucht von Tieren ist es oftmals notwendig, den Tieren eine Injektion zu verabreichen. Dies kann manuell mit einer herkömmlichen Spritze erfolgen. Bei größeren Betrieben, beispielsweise in der Geflügelhaltung, sind viele Tiere vorhanden, denen eine Injektion in möglichst kurzer Zeit verabreicht werden soll. Daher gibt es im Stand der Technik Injektionsvorrichtungen, mittels welchen eine Injektion an einem Tier schneller vorgenommen werden kann.

Die DE 10 2015 122 069 beschreibt beispielsweise eine Vorrichtung, bei der eine Anlageplatte mit einer anatomisch geformten Anlagefläche verschiebbar an einem Basiskörper gelagert ist. Das Tier, dem die Injektion verabreicht werden soll, wird gegen die Anlageplatte gedrückt, so dass diese in Richtung zum Basiskörper hin verschoben bis zu einer Endstellung verschoben wird, in der dann die Injektion verabreicht wird. Dazu wird eine Spritze mit einer Nadel durch eine Öffnung in der Anlagefläche hindurchbewegt und somit in das Tier eingestochen und in diesem eingestochenen Zustand wird dann die Injektion verabreicht. Danach wird die Spritze mit der Nadel wieder zurückgefahren, so dass die Nadel nicht mehr vor die Anlagefläche vorsteht.

Aufgabe der Erfindung ist es, eine verbesserte Vorrichtung zum Injizieren eines Fluids in ein Tier bereitzustellen.

Die Erfindung ist im Anspruch 1 definiert. Vorteilhafte Weiterbildungen sind in den abhängigen Ansprüchen angegeben.

Die erfindungsgemäße Vorrichtung zum nadellosen Injizieren eines Fluids in ein Tier kann eine Anlageeinrichtung, die eine geformte Anlagefläche aufweist, die entsprechend einem Körperteil des Tiers geformt ist, dem die Injektion verabreicht werden soll, aufweisen. Ferner kann die Vorrichtung einen ersten Injektor zum nadellosen Injizieren, der ein vorderes Abgabeende aufweist, das sich beim nadellosen Injizieren durch eine erste Durchgangsöffnung in der geformten Anlagefläche erstreckt und gegenüber der geformten Anlagefläche in einem Bereich angrenzend an die erste Durchgangsöffnung vorsteht, eine erste Messeinrichtung, die mindestens ein erstes Messsignal abgibt, um einen Kontakt des Tiers mit dem vorderen Abgabeende des ersten Injektors zu detektieren, und eine Steuereinheit, die ein nadelloses Injizieren mittels des ersten Injektors basierend auf dem mindestens einen ersten Messsignal aktiviert (bzw. auslöst oder ansteuert), umfassen.

Damit kann sicher und zuverlässig das gewünschte nadellose Injizieren bei einem Tier durchgeführt werden.

Die Steuereinheit kann insbesondere basierend auf dem mindestens einen ersten Messsignal entscheiden, ob ein Kontakt des Tieres mit dem vorderen Abgabeende des ersten Injektors vorliegt. Falls die Steuereinheit einen solchen Kontakt als gegeben ansieht, kann sie dann das nadellose Injizieren ansteuern.

Unter einem Kontakt zwischen dem Tier und dem vorderen Abgabeende des ersten Injektors wird insbesondere ein solcher Kontakt verstanden, der für ein nadelloses Injizieren vorliegen muss. Bevorzugt ist dies ein direkter Kontakt mit der Haut des Tieres.

Die Messeinrichtung kann so ausgebildet sein, dass sie laufend erste Messsignale abgibt oder dass sie ein erstes Messsignal bei Erreichen des Kontaktes von Tier und vorderem Abgabeende des ersten Injektors abgibt. Insbesondere kann die Messeinrichtung mindestens zwei unterschiedliche Messungen durchführen und die dabei erzeugten ersten Messsignale an die Steuereinheit übertragen.

Die Anlagefläche kann zwischen einer Grundposition und einer Injektionsposition verschiebbar gelagert sein, wobei sich das vordere Abgabeende des ersten Injektors in der Grundposition der Anlagefläche nicht durch die erste Durchgangsöffnung in der geformten Anlagefläche erstreckt und wohingegen sich das vordere Abgabeende des ersten Injektors in der Injektionsposition der Anlagefläche durch die erste Durchgangsöffnung in der geformten Anlagefläche erstreckt und gegenüber der geformten Anlagefläche in einem Bereich angrenzend an die erste Durchgangsöffnung vorsteht.

Die erste Messeinrichtung kann kontaktlos das Erreichen der Auslöselage detektieren.

Die Anlagefläche kann so gelagert sein, dass sich die Anlagefläche ohne dagegen gedrücktes Tier in der Grundposition befindet und dass die Anlagefläche erst bei Überschreiten einer vorbestimmten Kraft durch Andrücken des Tiers in die Injektionsposition bewegbar ist.

Insbesondere kann die Anlagefläche federvorgespannt in der Grundposition gehalten sein.

Die Vorrichtung zum nadellosen Injizieren eines Fluids in ein Tier kann eine erste Linearführung aufweisen, die den ersten Injektor trägt, entlang einer ersten Verschieberichtung zwischen einer vorderen Endlage und einer Auslöselage bewegbar lagert und in der vorderen Endlage federvorgespannt hält, wobei die Messeinrichtung bei Erreichen der Auslöselage durch den ersten Injektor ein erstes Messsignal abgibt. Die erste Linearführung ist bevorzugt so ausgebildet, dass sich das vordere Abgabeende des ersten Injektors in der vorderen Endlage nicht durch die erste Durchgangsöffnung in der geformten Anlagefläche erstreckt und nicht gegenüber der geformten Anlagefläche in einem Bereich angrenzend an die erste Durchgangsöffnung vorsteht. Alternativ ist es jedoch auch möglich, dass sich das vordere Abgabeende des ersten Injektors in der vorderen Endlage durch die erste Durchgangsöffnung in der geformten Anlagefläche erstreckt und gegenüber der geformten Anlagefläche in einem Bereich angrenzend an die erste Durchgangsöffnung vorsteht.

Die Anlageeinrichtung kann insbesondere eine Anlageplatte aufweisen, die die geformte Anlagefläche aufweist.

Die Anlageeinrichtung kann an einem Gehäuse der Vorrichtung positioniert oder gelagert sein. Dabei kann die Anlageeinrichtung so ausgebildet und am Gehäuse gelagert sein, dass die geformte Anlagefläche entlang der ersten Verschieberichtung in Richtung zur Auslöselage ortsfest ist. Insbesondere kann die Anlageeinrichtung austauschbar oder auswechselbar angeordnet sein.

Die erste Messeinrichtung kann so ausgebildet sein, dass sie kontaktlos das Erreichen der Auslöselage detektiert.

Natürlich ist es auch möglich, dass die erste Messeinrichtung mittels der Detektion eines Kontaktes das Erreichen der Auslöselage detektiert.

Die erste Linearführung kann einen entlang der Verschieberichtung verschiebbaren Schlitten, der den ersten Injektor trägt, aufweisen. Die erste Messeinrichtung kann einen Sensor zur Detektion der Auslöselage aufweisen. Insbesondere kann dazu die Position des Schlittens gemessen werden.

Der Sensor kann an einer Position in der Messanordnung angeordnet sein, die ortsfest relativ zu der Verschiebebewegung des Schlittens ist. Alternativ kann der Sensor am Schlitten selbst befestigt sein und sich somit mit dem Schlitten bewegen.

Bei dem Sensor kann es sich um einen Näherungssensor oder einen Abstandssensor handeln, der mittels Ultraschall, magnetisch, elektrisch, kapazitiv, induktiv, mechanisch und/oder optisch misst. Insbesondere kann es sich bei dem Sensor um einen Hall-Sensor handeln und somit um einen Sensor, der mittels eines Magneteffekts detektiert. Ferner kann z.B. ein Potenziometer oder ein Linearpotenziometer als Sensor eingesetzt werden, mit dem z.B. der Schlitten (oder ein mit dem Schlitten ortsfest verbundenes Element) bei Erreichen der Auslöselage mechanisch in Kontakt kommt. Dabei kann der Kontakt durchaus über eine gewisse Wegstrecke vorliegen, so dass eine genaue Messung der Auslöselage und/oder eine Kalibrierung zur Festlegung der Auslöselage möglich ist. Natürlich ist es auch möglich, den Sensor als Schalter auszubilden, mit dem z.B. der Schlitten (oder ein mit dem Schlitten ortsfest verbundenes Element) bei Erreichen der Auslöselage mechanisch in Kontakt kommt, wobei der Schalter somit nur eine Ja-Nein-Aussage liefert. Dabei bezeichnet die Ja-Aussage das Vorhandensein eines Kontaktes und somit das Erreichen der Auslöselage, wohingegen die Nein-Aussage das Nicht-Vorhandensein des Kontaktes bezeichnet.

Unter einem Näherungssensor wird hier insbesondere ein Sensor verstanden, der ein Schaltsignal ausgibt, nachdem der erste Injektor die Auslöselage erreicht. Unter einem Abstandssensor wird hier insbesondere ein Sensor verstanden, der ein Signal ausgibt, das proportional zum erfassten Abstand ist. Damit ist es leicht möglich, die Position der Auslöselage zu korrigieren und/oder individuell einzustellen.

Die erste Linearführung kann eine erste Feder aufweisen, die den ersten Injektor in der vorderen Endlage hält. Die erste Feder kann so ausgelegt sein, dass eine Kraft von 5 bis 10 Newton auf das vordere Abgabeende ausgeübt werden muss, um ein Verschieben des Injektors gegen die Federkraft der ersten Feder zu bewirken.

Der Abstand in Verschieberichtung zwischen der vorderen Endlage und der Auslöselage kann im Bereich von 5 bis 10 mm liegen. Somit muss durch Aufdrücken des Tieres an die Anlagefläche eine Verschiebung von 5 bis 10 mm erreicht werden. Wenn dies der Fall ist, wird dann das nadellose Injizieren durchgeführt. Damit kann z.B. sichergestellt werden, dass beim Huhn das vordere Abgabeende durch das Federkleid in Kontakt mit der Haut z.B. im Bereich des Brustmuskels gelangt.

Die erste Linearführung kann eine Anschlagfläche sowie eine am Schlitten gelagerte Stange aufweisen, wobei die Stange parallel zur ersten Verschieberichtung verschiebbar gelagert und mittels einer Feder zur Anlagefläche hin vorgespannt ist. Ein vorderes Ende der Stange kann bei Erreichen der Auslöselage an der Anschlagfläche anliegen, so dass die zweite Feder den Schlitten mit einer Kraft zur Anlagefläche hin beaufschlagt. Dies kann z.B. in vorteilhafter Weise dazu genutzt werden, eine zusätzliche Kraft (zusätzlich zur Kraft der ersten Feder) für das nadellose Injizieren bereitzustellen, um beispielsweise einen beim nadellosen Injizieren auftretenden Rückstoß zu kompensieren, so dass während des nadellosen Injizierens der Kontakt des vorderen Abgabeendes mit der Haut des Tieres sicher beibehalten wird.

Am vorderen Ende der Stange kann z.B. ein Magnet (bevorzug ein Dauermagnet) angeordnet sein, dessen Magnetfeld mittels des Sensors detektiert werden kann, um das Erreichen der Auslöselage festzustellen.

Insbesondere kann das Fluid in flüssiger Form bzw. als Flüssigkeit vorliegen. Bei dem zu injizierenden Fluid kann es sich um ein Medikament und/oder einen Impfstoff handeln. Insbesondere kann das Medikament und/oder der Impfstoff als Flüssigkeit verabreicht werden.

Bei der Vorrichtung kann sich das vordere Abgabeende des Injektors weiterhin durch die erste Durchgangsöffnung in der geformten Anlagefläche erstrecken und gegenüber der geformten Anlagefläche in einem Bereich angrenzend an die erste Durchgangsöffnung vorstehen, wenn der erste Injektor in der Auslöselage steht. Damit wird sichergestellt, dass ein direkter Kontakt zwischen dem vorderen Abgabeende und dem Bereich des Tiers, in den die Injektion ausgeführt werden soll, vorliegt.

Der erste Injektor kann insbesondere als Selbstfüllerspritze ausgebildet sein. Dazu kann der erste Injektor einen Zuführanschluss aufweisen, an den ein Fluidreservoir anschließbar ist, so dass nach einer nadellosen Injektion der erste Injektor wieder mit zu injizierendem Fluid gefüllt wird. Das Fluidreservoir kann, muss aber nicht, Teil der erfindungsgemäßen Vorrichtung zum nadellosen Injizieren sein.

Die erste Messeinrichtung kann so ausgebildet sein, dass sie eine auf den ersten Injektor ausgeübte Kraft durch Andrücken des Tiers an die Anlagefläche und damit gegen das vordere Abgabeende des ersten Injektors detektiert und entsprechende erste Messsignale erzeugt und an die Steuereinheit überträgt. Ein Überschreiten eines vorbestimmten Grenzwerts kann dann von der Steuereinheit als Kontakt zwischen dem Tier und dem vorderen Abgabeende des ersten Injektors bewertet werden, so dass ein nadelloses Injizieren angesteuert werden kann.

Ferner kann die erste Messeinrichtung so ausgebildet sein, dass sie eine auf die Anlagefläche ausgeübte Kraft durch Andrücken des Tiers an die Anlagefläche detektiert und entsprechende erste Messsignale erzeugt und an die Steuereinheit überträgt, die dann z.B. bei Überschreiten eines vorbestimmten Grenzwerts das nadellose Injizieren auslöst.

Ferner kann die Messeinrichtung einen Näherungssensor (beispielsweise einen kapazitiven Sensor) aufweisen, der das Vorhandensein des Tiers an der Anlagefläche detektiert und mindestens ein erstes Messsignal abgibt. Der Näherungssensor kann beispielsweise hinter der Anlagefläche positioniert sein, so dass kein direkter Kontakt zwischen dem Tier und dem Sensor möglich ist, der Sensor jedoch das Vorhandensein des Tiers an der Anlagefläche detektieren kann.

Die Steuereinheit ist insbesondere so ausgebildet, dass sie basierend auf einem einzigen ersten Messsignal oder mehreren ersten Messsignalen (die z.B. in der beschriebenen Art und Weise durch unterschiedliche Messmethoden erzeugt werden) das nadellose Injizieren mittels des ersten Injektors aktiviert bzw. auslöst oder ansteuert.

Der erste Injektor kann eine Kolben-Zylinder-Anordnung aufweisen, in der eine federvorgespannte Kolbenstange mit ihrem vorderen Ende in einem Zylinder zur Aufnahme des zu injizierenden Fluids geführt ist, wobei der Zylinder über ein erstes Rückschlagventil in eine Düse mündet. Ferner kann ein Zufuhranschluss vorgesehen sein, der über ein zweites Rückschlagventil mit dem Zylinder verbunden ist. An den Zuführanschluss kann ein Fluidreservoir angeschlossen sein.

Der Zylinder kann zusammen mit dem ersten Rückschlagventil und der Düse (und bevorzugt zusammen mit dem zweiten Rückschlagventil und dem Zufuhranschluss) als lösbarer Zylinderabschnitt ausgebildet sein, in dem am von der Düse abgewandten Ende eine Kolbenstangenführung lösbar angeordnet ist. Die Kolbenstangenführung kann eine aus Kunststoff hergestellte Führungsbuchse zur Führung der Kolbenstange sowie ein mit der Führungsbuchse verbundenes Fixierelement aus Metall aufweisen. Im eingebauten Zustand kann das Fixierelement im Zylinderabschnitt fixiert (z.B. eingeschraubt) sein, wobei das Fixierelement einen Eingriffsbereich für ein Werkzeug aufweist, in den ein Werkzeug eingesetzt werden kann, um dann mittels des Werkzeuges das Fixierelement aus dem Zylinderabschnitt zu lösen (z.B. herauszuschrauben), wenn der Zylinderabschnitt vom restlichen Injektor gelöst ist.

Damit kann in vorteilhafter Weise einerseits eine gute Führung der Kolbenstange mittels der aus Kunststoff hergestellten Führungsbuchse bereitgestellt werden. Andererseits kann die Führungsbuchse zu Wartungszwecken leicht aus- und eingebaut werden ohne sie zu beschädigen, da dazu der Eingriff zwischen dem Werkzeug und dem aus Metall hergestellten Fixierelement erfolgt.

Der Eingriffsbereich des Fixierelementes ist dabei bevorzugt so ausgebildet, dass sich durch diesen die Kolbenstange hindurch erstrecken kann, ohne ihn zu berühren.

Die Verbindung zwischen der Führungsbuchse und dem Fixierelement ist bevorzugt werkzeuglos trennbar ist.

Dazu kann beispielsweise die Verbindung zwischen der Führungsbuchse und dem Fixierelement als formschlüssige Verbindung ausgebildet sein.

Die Führungsbuchse kann ein Durchgangsloch zur Führung der Kolbenstange aufweisen. Das Durchgangsloch kann z.B. hohlzylinderförmig ausgebildet sein. Der Querschnitt des Durchgangsloches kann z.B. kreisförmig sein.

Die formschlüssige Verbindung zwischen der Führungsbuchse und dem Fixierelement kann insbesondere in einer Richtung parallel zur Längsrichtung des Durchgangsloches ausgebildet sein. Ferner kann, muss aber nicht, die formschlüssige Verbindung zwischen der Führungsbuchse und dem Fixierelement quer zur Längsrichtung des Durchgangsloches ausgebildet sein, wobei in diesem Fall bevorzugt eine seitliche Öffnung oder Ausnehmung vorliegt, in der keine formschlüssige Verbindung vorliegt, so dass durch diese Öffnung oder Ausnehmung die Führungsbuchse vom Fixierelement gelöst bzw. getrennt werden kann.

Die Führungsbuchse und das Fixierelement sind bevorzugt lösbar miteinander verbunden. Hierunter wird insbesondere verstanden, dass die Verbindung von Führungsbuchse und Fixierelement ohne Beschädigung von Führungsbuchse oder Fixierelement gelöst werden kann.

Die Führungsbuchse kann an ihrer Außenseite eine erste Ringnut aufweisen. Das Fixierelement kann einen seitlich offenen Kragen mit einer seitlichen Öffnung aufweisen, wobei über die seitliche Öffnung des Kragens die Führungsbuchse in den Kragen hinein- und aus dem Kragen herausgeschoben werden kann. Dies ist also eine Bewegung senkrecht zur Längserstreckung der Führungsbuchse.

Somit kann auch die Führungsbuchse, soweit notwendig ist, ausgetauscht werden.

Die Führungsbuchse kann an ihrer Außenseite eine zweite Ringnut aufweisen, in der eine Dichtung (z.B. eine Ring-Dichtung oder eine O-Ring-Dichtung) sitzt.

Des Weiteren kann die Führungsbuchse eine Ringnut an ihrer Innenseite aufweisen, an der eine zweite Dichtung (z.B. eine zweite Ring-Dichtung oder eine zweite O-Ring-Dichtung) sitzt.

Die erfindungsgemäße Vorrichtung kann einen zweiten Injektor sowie eine zweite Messeinrichtung aufweisen, die in gleicher Weise ausgebildet sind wie der erste Injektor und die erste Messeinrichtung. Der zweite Injektor kann somit ein vorderes Abgabeende aufweisen, das sich beim nadellosen Injizieren durch eine zweite Durchgangsöffnung in der geformten Anlagefläche erstreckt und gegenüber der geformten Anlagefläche in einem Bereich angrenzend an die zweite Durchgangsöffnung vorsteht. Ferner kann die zweite Messeinrichtung mindestens ein zweites Messsignal abgeben, um einen Kontakt des Tiers mit dem vorderen Abgabeende des zweiten Injektors zu detektieren, wobei die Steuereinheit ein nadelloses Injizieren mittels des zweiten Injektors basierend auf dem mindestens einen zweiten Messsignal aktiviert.

Ferner kann eine zweite Linearführung für den zweiten Injektor vorgesehen sein, die in gleicher Weise wie die erste Linearführung für den ersten Injektor ausgebildet ist.

Dieser zweite Injektor kann somit entlang einer zweiten Verschieberichtung zwischen einer vorderen Endlage und einer Auslöselage bewegbar gelagert und in der vorderen Endlage federvorgespannt gehalten sein. Die nadellose Injektion kann dann in gleicher Weise wie mittels des ersten Injektors erfolgen, wenn mittels der zweiten Messeinrichtung die Bewegung des zweiten Injektors bis zur Auslöselage detektiert wird.

Die erste und zweite Verschieberichtung können zueinander parallel sein oder einen Winkel miteinander einschließen, der im Bereich zwischen 0° und 45° liegt und bevorzugt kleiner als 40°, 35°, 30° oder 25° und bevorzugt größer als 5°, 10°, 15° oder 20° ist.

Damit können dem Tier gleichzeitig zwei verschiedene Fluide, wie z.B. Medikamente und/oder Impfstoffe nadellos injiziert werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbeispiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:

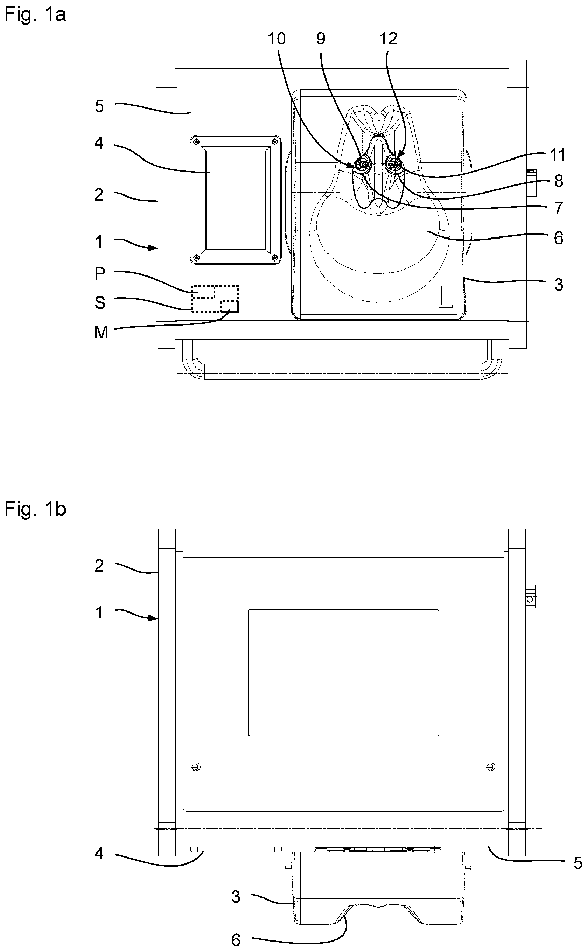

- Fig. 1a: eine Vorderansicht einer Ausführungsform der Vorrichtung 1 zum nadellosen Injizieren eines Fluids;
- Fig. 1: b eine Draufsicht der Vorrichtung 1 von Fig. 1;
- Fig. 1c: eine Vorderansicht der Vorrichtung 1 von Fig. 1 mit entfernter Anlageplatte 3;
- Fig. 2: eine Darstellung der Rückseite der Anlageplatte 3;
- Fig. 3: eine isometrische Ansicht des Injektors 10 zusammen mit der Linearführung 15;
- Fig. 4: eine Schnittansicht der Zylinder-Kolben-Anordnung 16, bei der die Kolbenstange 26 in ihrer vorderen Endposition ist;
- Fig. 5: eine Schnittansicht der Zylinder-Kolben-Anordnung 16, bei der die Kolbenstange 26 in ihrer hinteren Endposition ist;
- Fig. 6: eine isometrische Explosionsdarstellung des Injektors 10 zusammen mit der Linearführung 15;
- Fig. 7: eine Draufsicht des Injektors 10 mit der Linearführung 15;
- Fig. 8: eine Schnittansicht des Injektors 10 mit der Linearführung 15 entlang der Schnittlinie A-A in Fig. 7;
- Fig. 9: eine isometrische Explosionsdarstellung der Linearführung 15;
- Fig. 10: eine Draufsicht der Linearführung 15;
- Fig. 11: eine Schnittansicht der Linearführung 15 entlang der Schnittlinie B-B von Fig. 10;
- Fig. 12: eine Seitenansicht des Zylinderabschnitts 60;
- Fig. 13: eine Schnittansicht des Zylinderabschnitts 60 entlang der Schnittlinie C-C in Fig. 12;
- Fig. 14: eine isometrische Explosionsdarstellung des Zylinderabschnitts 60;
- Fig. 15: eine Seitenansicht der Kolbenstangenführung 63;
- Fig. 16: eine Schnittansicht der Kolbenstangenführung 63 entlang der Schnittlinie E-E in Fig. 15;
- Fig. 17 und 18: isometrische Explosionsdarstellungen der Kolbenstangenführung 63;

- Fig. 19: eine isometrische Ansicht des Injektors 10 zusammen mit der Linearführung 15 einer weiteren Ausführungsform der Vorrichtung 1 zum nadellosen Injizieren eines Fluids;
- Fig. 20: eine isometrische Explosionsdarstellung des Injektors 10 zusammen mit der Linearführung 15 von Fig. 19;
- Fig. 21: eine Draufsicht des Injektors 10 mit der Linearführung 15 von Fig. 19;
- Fig. 22: eine Schnittansicht des Injektors 10 mit der Linearführung 15 entlang der Schnittlinie F-F in Fig. 21;
- Fig. 23: eine isometrische Explosionsdarstellung der Linearführung 15 von Fig. 19;
- Fig. 24: eine Draufsicht der Linearführung 15 von Fig. 23;
- Fig. 25: eine Schnittansicht der Linearführung 15 entlang der Schnittlinie G-G von Fig. 24;

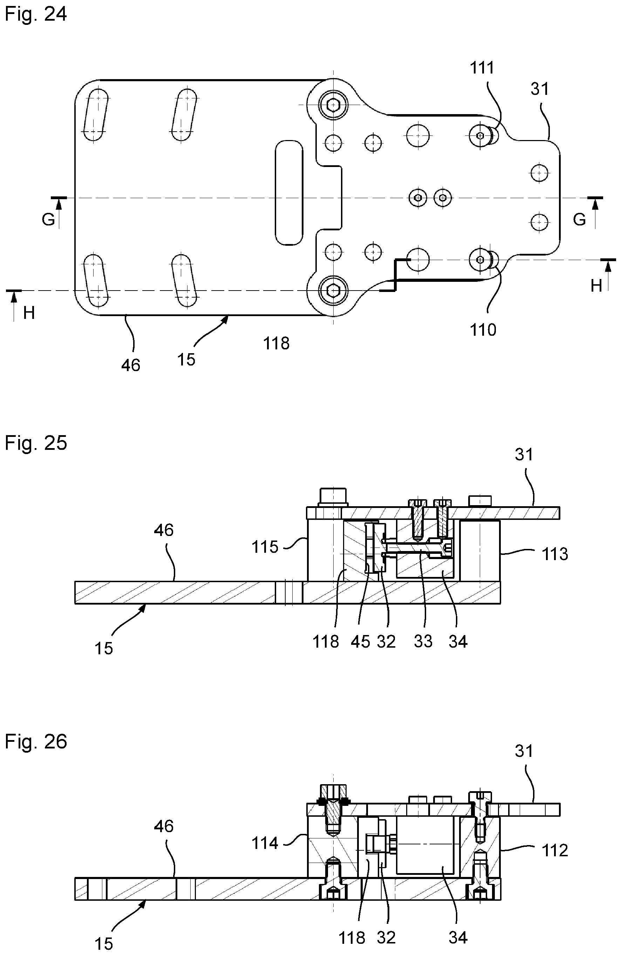

- Fig. 25: eine Schnittansicht der Linearführung 15 entlang der Schnittlinie H-H von Fig. 24,
- Fig. 26: eine Vorderansicht einer weiteren Ausführungsform der Vorrichtung 1 zum nadellosen Injizieren eines Fluids;

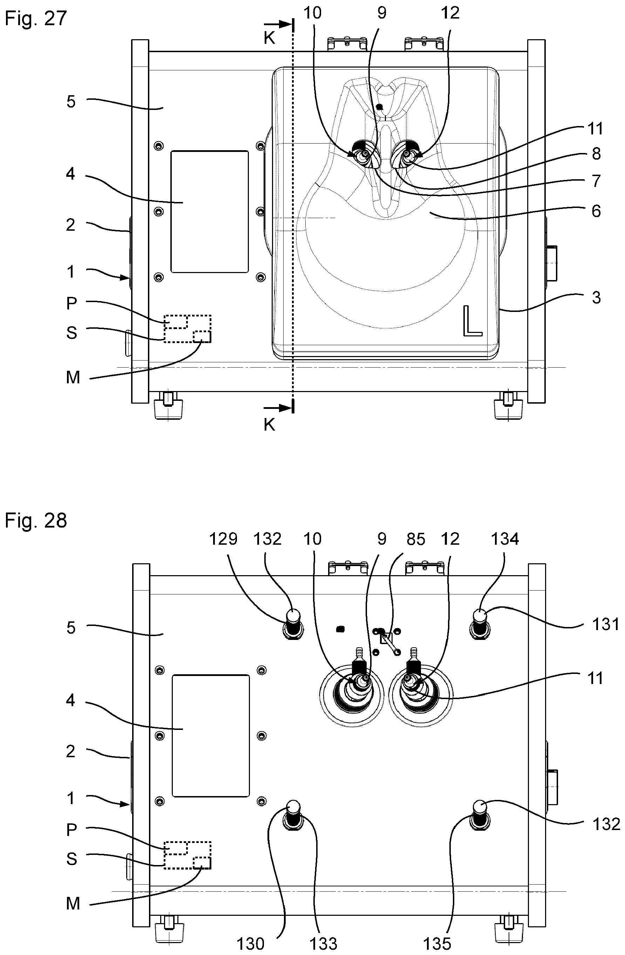

- Fig. 27: eine Vorderansicht der Vorrichtung 1 von Fig. 26 mit entfernter Anlageplatte 3; und
- Fig. 28: eine Schnittansicht der Vorrichtung 1 entlang der Schnittlinie K-K von Fig. 27.

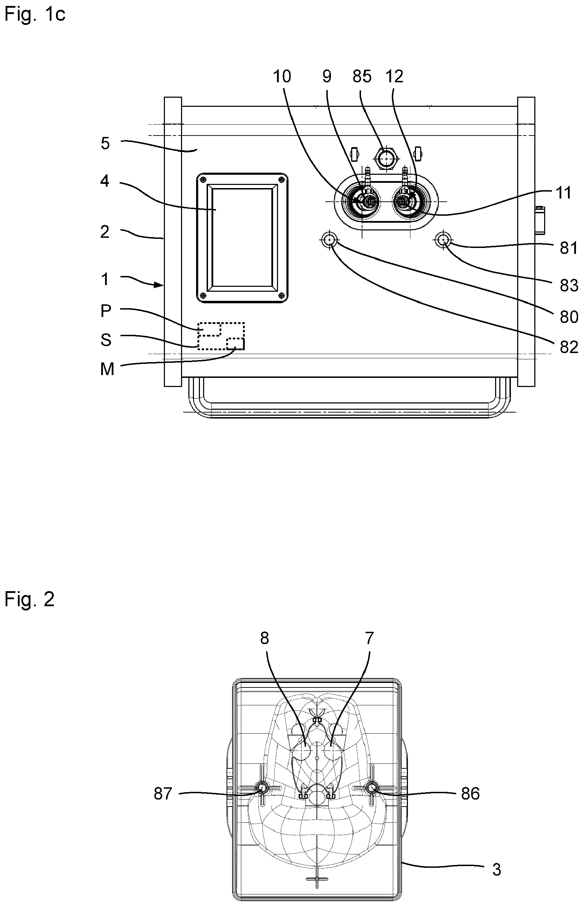

Bei der in Fig. 1a - 1c und 2 dargestellten Ausführungsform umfasst die erfindungsgemäße Vorrichtung 1 zum nadellosen Injizieren eines Fluids ein Gehäuse 2 mit einer Anlageplatte 3 und einer Anzeige 4 an einer Vorderseite 5 des Gehäuses 2. Ferner ist eine im Gehäuse 2 angeordnete Steuereinheit S mit einem Prozessor P und einem Speicher M zur Steuerung der Vorrichtung 1 vorgesehen, die lediglich in Fig. 1a und 1c schematisch gestrichelt dargestellt ist.

Die Vorrichtung 1 zum nadellosen Injizieren eines Fluids dient zur Verabreichung von Injektionen an einem Tier. Mit der dargestellten Ausführungsform können beispielsweise einem Huhn Impfstoffe und/ oder Medikamente intramuskulär verabreicht werden.

Dazu weist die Anlageplatte 3 eine geformte Anlagefläche 6 auf, deren Form so gewählt ist, dass sie anatomisch an das Tier angepasst ist, dem der Impfstoff oder das Medikament verabreicht werden soll. Bei der hier beschriebenen Ausführungsform ist die geformte Anlagefläche 6 entsprechend dem Brustbereich eines Huhns geformt. Insbesondere kann die Anlageplatte 3 abnehmbar an der Vorderseite 5 vorgesehen sein. Es können somit nacheinander verschiedene Anlageplatten 3 angebracht werden, die z.B. an die zu behandelnde Tierart oder -rasse, dem Alter der Tiere und/oder ihrer Größe angepasst sind.

Wie insbesondere in Fig. 1a gut erkennbar ist, sind in der geformten Anlagefläche 6 eine erste und eine zweite Durchgangsöffnung 7, 8 ausgebildet. Durch die erste Durchgangsöffnung 7 erstreckt sich ein vorderes Abgabeende 9 eines ersten nadellosen Injektors 10 und durch die zweite Durchgangsöffnung 8 erstreckt sich ein vorderes Abgabeende 11 eines zweiten nadellosen Injektors 12. Damit stehen die beiden Abgabeenden 9, 11 gegenüber den den beiden Durchgangsöffnungen 7, 8 direkt umgebenden Bereichen der geformten Anlagefläche 6 vor.

Wie insbesondere der Darstellung in Fig. 1c zu entnehmen ist, sind an der Vorderseite 5 des Gehäuses 2 ein erstes und ein zweites zylinderförmiges Stützelement 80, 81 ausgebildet, die sich im Wesentlichen senkrecht zur Vorderseite 5 erstrecken. Das erste zylinderförmige Stützelement 80 umfasst einen ersten Kraftsensor 82 und das zweite zylinderförmige Stützelement 81 umfasst einen zweiten Kraftsensor 83. Die Kraftsensoren können beispielsweise jeweils als eine aus dem Stand der Technik bekannte Wägezelle ausgebildet sein.

Ferner ist noch ein Näherungssensor 85 an der Vorderseite 5 angeordnet. Der Näherungssensor 85 kann beispielsweise als kapazitiver Sensor 85 ausgebildet sein.

Die beiden zylinderförmigen Stützelemente 80 und 81 dienen zur Aufnahme und Halterung der Anlageplatte 3, die dazu auf ihrer Rückseite, wie in Fig. 2 gezeigt ist, zwei zylinderförmige Hohlräume 86, 87 aufweist, so dass die Anlageplatte 3 auf die beiden Stützelemente 80, 81, die in die zylinderförmigen Hohlräume 86, 87 eintauchen, aufgeschoben werden kann.

Wie der Darstellung in Fig. 1c besonders gut zu entnehmen ist, sind die beiden zylinderförmigen Stützelemente 80, 81 und somit ihre Kraftsensoren 82, 83 links und rechts der beiden Abgabeenden 9, 11 der beiden Injektoren 10, 12 angeordnet. Der Näherungssensor 85 ist oberhalb der beiden Abgabeenden 9, 11 angeordnet, so dass die beiden Kraftsensoren 82 und 83 zusammen mit dem Näherungssensor 85 ein Dreieck aufspannen, das hier ein gleichschenkliges Dreieck ist.

Durch die Anordnung der beiden Kraftsensoren 82 und 83 kann die Kraft gemessen werden, mit der das Tier (hier das Huhn) gegen die Anlageplatte 3 gedrückt wird. Insbesondere kann detektiert werden, ob das Huhn schräg bzw. schief dagegen gedrückt wird, da in diesem Fall die beiden Kraftsensoren 82 und 83 deutlich unterschiedliche Messwerte anzeigen. Der Näherungssensor 85 dient auch zur Messung, ob ein Huhn nahe genug an der Anlageplatte 3 anliegt. Sowohl die Kraftsensoren 82 und 83 als auch der Näherungssensor 85 können Messsignale abgeben und an die Steuereinheit S weiterleiten. Die Steuereinheit S kann dann diese Messsignale auswerten und entscheiden, ob das Huhn bestimmungsgemäß an der Anlageplatte 3 anliegt.

Wie nachfolgend noch detailliert beschrieben wird, sind die beiden nadellosen Injektoren 10, 12 jeweils auf einer Linearführung verschiebbar gelagert, wobei eine Injektion erst dann ausgelöst wird, wenn der entsprechende nadellose Injektor 10, 12 um eine vorbestimmte Strecke verschoben wurde. Dieses Verschieben wird bei bestimmungsgemäßem Gebrauch der Vorrichtung 1 dadurch erreicht, dass das Huhn mit seinem Brustbereich gegen die geformte Anlagefläche 6 gedrückt wird, so dass dadurch die vorderen Abgabeenden 9, 11 durch das Federkleid in Kontakt mit dem Brustmuskel (bzw. dem entsprechenden Hautbereich) kommen und somit die vorbestimmte Wegstrecke nach hinten geschoben werden. Damit wird sichergestellt, dass ein direkter Kontakt der Abgabeenden 9, 11 mit dem entsprechenden Hautbereich am Brustmuskel vorliegt und dann die nadellose Injektion ausgelöst und erfolgreich durchgeführt werden kann.

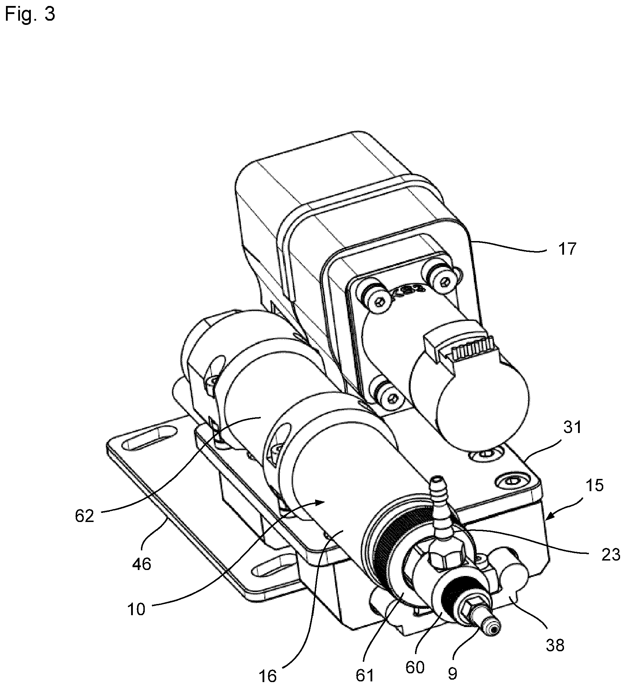

Nachfolgend wird der erste nadellose Injektor 10 zusammen mit der Linearführung unter Bezugnahme auf Fig. 3 bis 11 detaillierter beschrieben, wobei der zweite nadellose Injektor 12 zusammen mit der Linearführung in gleicher Weise ausgebildet ist. Der erste nadellose Injektor 10 wird im Weiteren nur nadelloser Injektor 10 genannt und umfasst eine Kolben-Zylinder-Anordnung 16 sowie eine Spannvorrichtung 17, die zusammen auf einer Linearführung 15 befestigt sind (Fig. 3).

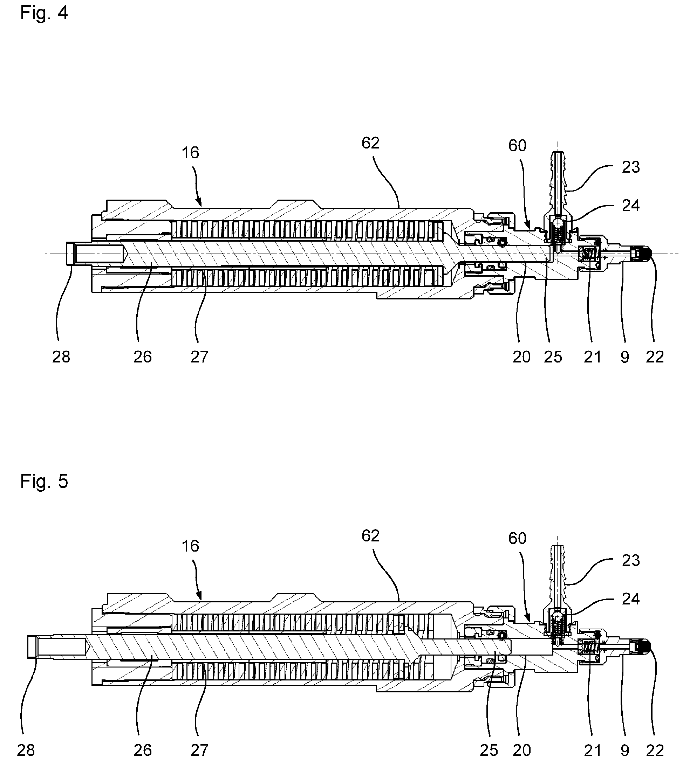

Wie insbesondere der Schnittdarstellung in Fig. 4 zu entnehmen ist, weist die Kolben-Zylinder-Anordnung 16 einen Zylinder 20 auf, der über ein erstes Rückschlagventil 21 mit dem vorderen Abgabeende 9, das eine Düse 22 zur Abgabe des Fluids umfasst, in Fluidverbindung steht. Das erste Rückschlagventil 21 ist somit so ausgebildet, dass eine Abgabe von Fluid aus dem Zylinder 20 über das erste Rückschlagventil 21 und die Düse 22 ermöglicht wird. Ein Ansaugen von Luft oder Flüssigkeit über die Düse 22 und über das erste Rückschlagventil 21 ist nicht möglich, da in dieser Richtung das erste Rückschlagventil 21 schließt.

Ferner ist ein Fluidanschluss 23 vorgesehen, der über ein zweites Rückschlagventil 24 in Fluidverbindung mit dem Zylinder 20 steht, so dass über den Fluidanschluss 23 Fluid in den Zylinder 20 gelangen kann. In der entgegengesetzten Richtung (also vom Zylinder 20 zum Fluidanschluss 23) sperrt das zweite Rückschlagventil 24. Das zweite Rückschlagventil 24 kann somit als Einlassventil und das erste Rückschlagventil 21 kann als Auslassventil bezeichnet werden.

In dem Zylinder 20 ist ein vorderes Ende 25 einer Kolbenstange 26 geführt, so dass das vordere Ende 25 als Kolben wirkt. Die Kolbenstange 26 ist durch eine Spiralfeder 27 zum vorderen Abgabeende 9 hin gespannt und weist ein nach hinten vorstehendes Ende 28 auf. Die Kolben-Zylinder-Anordnung 16 ist somit als Selbstfüllerspritze ausgebildet, da eine Bewegung der Kolbenstange 26 von ihrer in Fig. 4 gezeigten vorderen Endposition gegen die Federkraft der Spiralfeder 27 nach links in Fig. 4 zu einem Unterdruck im Zylinder 20 führt, wodurch das zweite Rückschlagventil 24 öffnet und Fluid eines mit dem Fluidanschluss 23 verbundenes Reservoir in den Zylinder 20 gesaugt wird. Wenn die Kolbenstange 26 in ihrer hinteren Endposition (Fig. 5) angekommen ist, schließt das zweite Rückschlagventil 24 und ist der Zylinder 20 mit Fluid gefüllt. Die Kolben-Zylinder-Anordnung 16 ist somit in einem vorgespannten Zustand. Wenn die Kolbenstange 26 freigegeben wird, wird diese durch die Spiralfeder 27 zum vorderen Abgabeende 9 hin beschleunigt, so dass ein Überdruck im Zylinder 20 aufgebaut wird, wodurch das erste Rückschlagventil 21 öffnet und das Fluid über die Düse 22 zur nadellosen Injektion abgegeben wird. Diese Bewegung der Kolbenstange 26 endet, wenn sie ihre vordere Endposition erreicht (Fig. 4). Ein erneutes Bewegen der Kolbenstange 26 entgegen der Federkraft der Spiralfeder 27 zur hinteren Endposition führt dann wieder zum Füllen des Zylinders 20 mit Fluid.

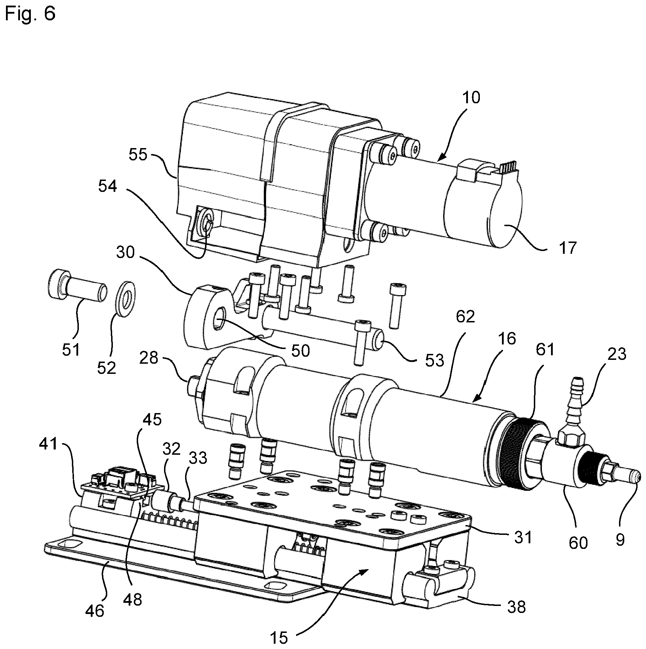

Um diese beschriebene Kolbenbewegung zum Füllen durchzuführen, ist am hinteren Ende 28 der Kolbenstange ein Mitnehmer 30 (Fig. 6-8) befestigt, der mittels der Spannvorrichtung 17 so bewegt werden kann, dass das beschriebene Spannen der Kolbenstange 26 einschließlich des Füllens des Zylinders 20 mit Fluid erfolgt, der vorgespannte Zustand der Kolben-Zylinder-Anordnung 16 so lange aufrecht erhalten wird, bis das Freigeben der Kolbenstange 26 zum nadellosen Injizieren durchgeführt wird. Die Befestigung des Mitnehmers 30 am hinteren Ende 28 erfolgt hier durch eine Durchgangsbohrung 50 im Mitnehmer 30 mittels einer Schraube 51 und einer Unterlegscheibe 52.

Zur Durchführung einer solchen Spannbewegung kann die Spannvorrichtung 17 z.B. einen Motor aufweisen, der eine Rampenbahn, die eine einzige Windung aufweist, in Drehung um eine Achse parallel zur Längsachse der Kolbenstange 26 versetzt. Eine drehbare Walze des Mitnehmers 30 steht in Kontakt mit der Rampenbahn, wobei der Mitnehmer 30 aufgrund einer am Mitnehmer 30 befestigten Führungsstange 53, die in einer Durchgangsöffnung 54 am Motorgehäuse 55 geführt ist, so in der Spannvorrichtung 17 gelagert ist, dass er nur parallel zur Längsachse der Kolbenstange 26 bewegbar ist. Eine Drehbewegung der Rampenbahn wird somit in eine translatorische Bewegung des Mitnehmers 30 parallel zur Längsachse der Kolbenstange 26 umgesetzt. Solche Spannvorrichtungen für Vorrichtungen zum nadellosen Applizieren sind bekannt. Es wird hier insbesondere auf die DE 10 2019 123 730 A1 Bezug genommen. Es kann dabei insbesondere auf Fig. 6 bis 10 und die Beschreibung in den Absätzen 75 bis 83 verwiesen werden.

Durch die vom Motor verursachte Drehung der Rampenbahn wird somit eine Bewegung der Kolbenstange 26 zur hinteren Endposition durchgeführt. Die Rampenbahn weist ein oberes Plateau auf, das der hinteren Endposition der Kolbenstange 26 entspricht, und wird in dieser Drehstellung angehalten, wodurch der vorgespannte Zustand der Kolben-Zylinder-Anordnung 16 gemäß Fig. 5 vorliegt.

Das obere Plateau der Rampenbahn ist über eine Sprungkante mit einem unteren Plateau der Rampenbahn verbunden. Wenn die Rampenbahn weiter gedreht wird, um eine nadelloses Injizieren durchzuführen, läuft die Walze über die Sprungkante, so dass keine Kraft zur Spannung der Feder 27 mehr vorliegt, wodurch die Kolbenstange 26 schlagartig zum vorderen Abgabeende 9 hin beschleunigt wird. Ein Drehen der Rampenbahn bis zum oberen Plateau führt dann zu einem nächsten Füllen des Zylinders.

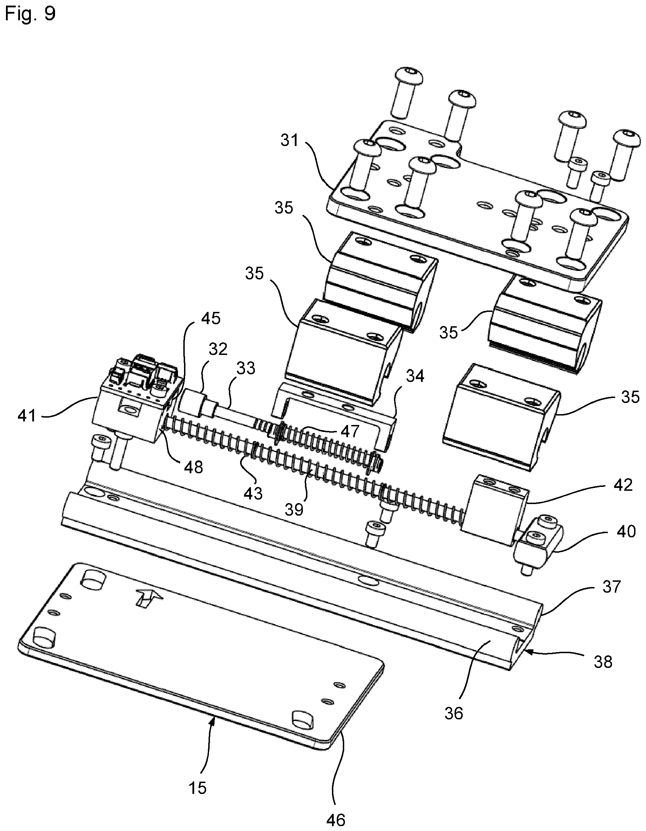

Wie insbesondere Fig. 3 und 6 bis 8 zu entnehmen ist, ist die Kolben-Zylinder-Anordnung 16 zusammen mit der Spannvorrichtung 17 auf einer Schlittenplatte 31 der Linearführung 15 montiert. Ferner ist an der Schlittenplatte 31 ein Magnet 32 über eine Haltestange 33 und einen Halter 34 an der Schlittenplatte 31 montiert (Fig. 9-11). Die Schlittenplatte 31 ist über vier Führungsblöcke 35 auf zwei Führungsschienen 36, 37 einer Führungsbasis 38 geführt, so dass nur eine Bewegung der Schlittenplatte 31 in Längsrichtung (nachfolgen auch Verschieberichtung genannt) der Führungsschienen 36, 37 möglich ist. Zusätzlich ist die Schlittenplatte 31 in Richtung zur Anlageplatte 3 der Vorrichtung 1 hin vorgespannt und steht dadurch in einer vorderen Endlage, wenn keine externe Kraft auf das vordere Abgabeende 9 des Injektors 10 ausgeübt wird. Dazu ist eine Führungsstange 39 zwischen zwei Stangenhaltern 40 und 41 vorgesehen, auf der ein Federanschlag 42 geführt ist, der seinerseits mit der Schlittenplatte 31 verbunden ist und mittels einer Feder 43 zur Anlageplatte 3 hin vorgespannt ist. Die in den Figuren gezeigten Schrauben zum Verbinden der Teile werden nicht detailliert beschrieben. Ihre Funktionen ergeben sich unmittelbar aus den Figuren.

Am zweiten Stangenhalter 41 ist ein Sensor 45 (hier ein Hall-Sensor 45) angeordnet, der benutzt wird, um den Abstand zum Magneten 32 zu detektieren.

Die Haltestange 33 ist im Halter 34 in der Verschieberichtung verschiebbar gelagert und mittels einer zweiten Feder 47 parallel zur Verschieberichtung zur Anlagefläche 6 vorgespannt.

Die Führungsbasis 38 ist auf einer Grundplatte 46 befestigt, die ihrerseits im Gehäuse 2 montiert ist.

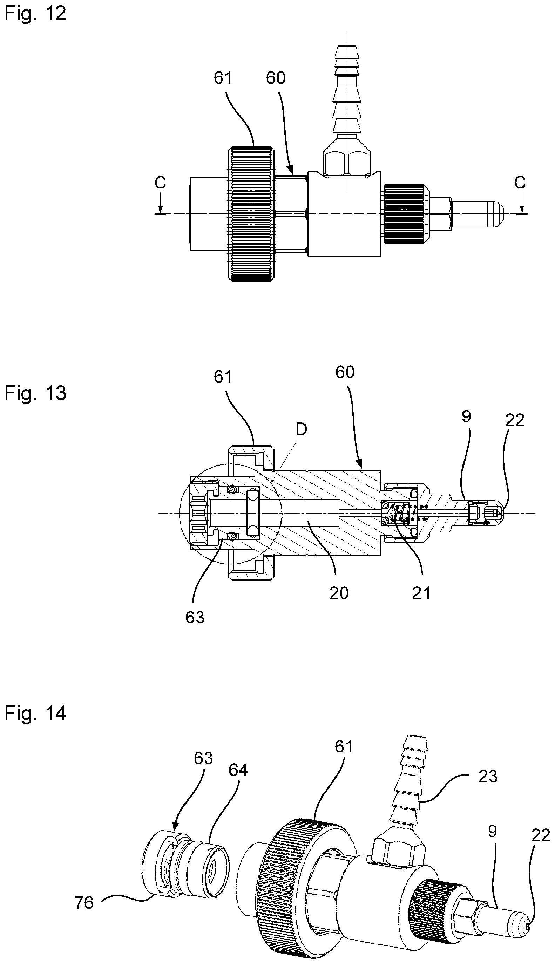

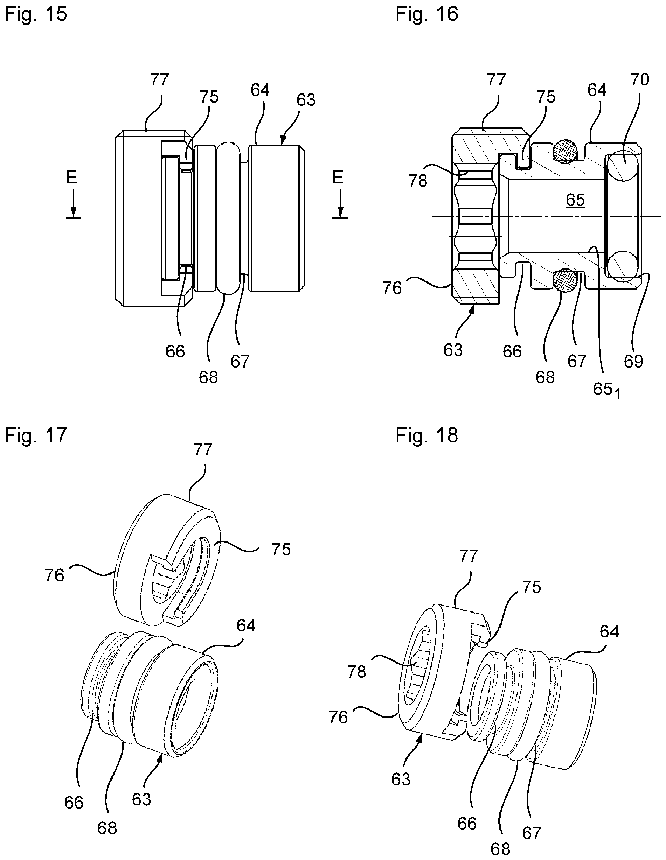

Der Zylinder 20 mit dem vorderen Abgabeende 9 und dem Fluidanschluss 23 ist als lösbarer Zylinderabschnitt 60 (Fig. 12 und 13) ausgebildet, der mit einer Überwurfmutter 61 (Fig. 3 bis 8) an einem hinteren Injektorabschnitt 62 befestigt ist. Zum Lösen des Zylinderabschnitts 60 muss lediglich die Anlageplatte 3 abgenommen werden, die Überwurfmutter 61 gelöst werden und dann kann der Zylinderabschnitt 60 vom hinteren Injektorabschnitt 62 abgezogen werden. Wie insbesondere in der Schnittdarstellung in Fig. 13 ersichtlich ist, weist der Zylinderabschnitt 60 eine Kolbenstangenführung 63 (Fig. 14 bis 16) auf, die ihrerseits lösbar im Zylinderabschnitt 60 befestigt ist. Die Kolbenstangenführung 63 umfasst eine Führungsbuchse 64, die zur Bereitstellung der notwendigen Gleiteigenschaften für die Kolbenstange 26 aus Kunststoff gebildet ist und die ein Durchgangsloch 65 aufweist, dessen Innenwandung 65₁ zur Führung des vorderen Kolbenstangenabschnitts 25 dient. An ihrer Außenseite weist die Führungsbuchse 64 eine erste Ringnut 66 sowie eine zweite Ringnut 67 auf. In der zweiten Ringnut 67 sitzt eine erste O-Ring-Dichtung 68. Ferner umfasst die Führungsbuchse 64 eine dritte Ringnut 69 an der Innenseite. In der dritten Ringnut 69 sitzt eine zweite O-Ring-Dichtung 70.

Da die O-Ring-Dichtungen 68 und 70 verschleißen, müssen sie regelmäßig ausgetauscht werden. Dazu ist es notwendig, dass die Kolbenstangenführung 63 aus dem Zylinderabschnitt 60 entnommen werden kann. Da die Führungsbuchse 64 relativ stark im Zylinderabschnitt 66 fixiert ist, um einen dauerhaften Betrieb zu gewährleisten, würde ein direktes Angreifen an die Führungsbuchse 64 mit einem Werkzeug zum Entnehmen der Führungsbuchse 64 zu ihrer Beschädigung führen.

Daher sitzt die Führungsbuchse 64 mit ihrer ersten Ringnut 66 in einem seitlich offenen Kragen 75 eines Fixierelements 76 mit einem Außengewinde 77 und einem Innensechskant 78. In der entsprechenden Aufnahme des Zylinderabschnitts 60 ist ein zu dem Außengewinde 77 passendes Innengewinde vorgesehen, so dass mit einem entsprechenden Werkzeug (z.B. Sechskantschlüssel), der in den Innensechskant 78 eingreift, das Fixierelement 76 in den Zylinderabschnitt 60 eingeschraubt und auch wieder ausgeschraubt werden kann. Sowohl das Fixierelement 76 als auch der Zylinderabschnitt 60 sind bevorzugt aus Metall gebildet. Sobald das Fixierelement 76 ausgeschraubt ist, kann damit die gesamte Kolbenstangenführung 63 entnommen werden, so dass die O-Ring-Dichtungen 68 und 70 getauscht werden können.

Da die Führungsbuchse 64 in dem seitlich offenen Kragen 75 sitzt, liegt in Längsrichtung des Durchgangslochs 65 eine formschlüssige Verbindung vor. In einer Richtung quer zur Längsrichtung des Durchgangslochs 65 ist die formschlüssige Verbindung nur durch den offenen Bereich des Kragens 75 seitlich aufgehoben, so dass die Führungsbuchse durch den offenen Bereich des Kragens 75 seitlich und somit quer zur Längsrichtung des Durchgangslochs 65 herausgeschoben werden kann. Somit ist es auch möglich, die Führungsbuchse 65 in der beschriebenen Art und Weise vom Fixierelement 76 zu lösen und dann die O-Ring-Dichtungen 68 und 70 auszuwechseln. Die Führungsbuchse 64 mit den ausgetauschten O-Ring-Dichtungen 68 und 70 kann dann wieder seitlich in den Kragen 75 eingeschoben und die gesamte Kolbenstangenführung 63 kann dann wiederum in der beschriebenen Art und Weise in den Zylinderabschnitt 60 eingeschraubt werden.

Somit können die Verschleißteile (hier die O-Ring-Dichtungen 68, 70) leicht ausgetauscht werden, ohne dass dabei die Führungsbuchse 64 beschädigt wird.

Ferner ist es möglich, die Führungsbuchse 64 selbst auszuwechseln, falls dies notwendig werden sollte. Die ausgewechselte Führungsbuchse 64 kann dann mittels des Fixierelements 76 ohne Beschädigung im Zylinderabschnitt 60 fixiert werden.

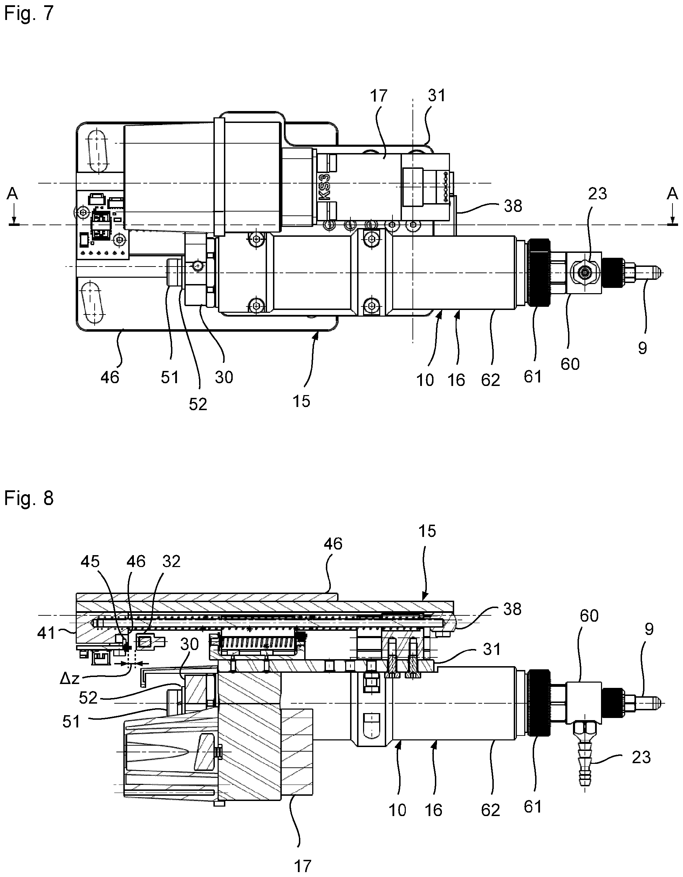

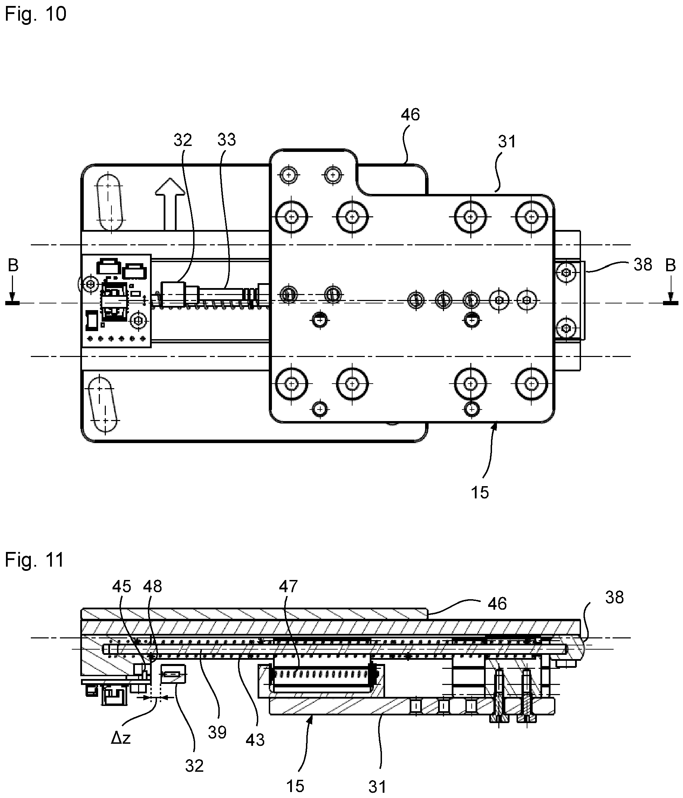

Beim bestimmungsgemäßen Gebrauch der Vorrichtung 1 zum nadellosen Injizieren eines Fluids steht der nadellose Injektor 10 aufgrund der Linearführung 15 (wegen der ersten Feder 43) federvorgespannt in seiner vorderen Endlage, in der sich das vordere Abgabeende 9 durch die erste Durchgangsöffnung 7 hindurch erstreckt. Wird nun das Tier (hier ein Huhn mit seinem Brustbereich) gegen die geformte Anlagefläche 6 gedrückt, kommt dadurch das vordere Abgabeende 9 durch das Federkleid in Kontakt mit dem Brustmuskel (bzw. dem entsprechenden Hautbereich). Aufgrund der Linearführung 15 führt ein Druck auf das vordere Abgabeende 9 des nadellosen Injektors 10 zu einer Bewegung des nadellosen Injektors 10 mit der Schlittenplatte 31 in Richtung zum zweiten Stangenhalter 41 gegen die Kraft der ersten Feder 43. Wenn der Magnet 32 in Kontakt mit dem zweiten Stangenhalter 41 (bzw. einer Anschlagfläche 48 des zweiten Stangenhalters 41) kommt, befindet sich der nadellose Injektor 10 in seiner Auslöselage. Der dabei zurückgelegte Weg Δz zwischen dem Magneten 32 und der Anschlagfläche 48 des zweiten Stangenhalters 41 (Fig. 8 und 11) kann im Bereich von 5 - 10 mm liegen. Ferner wurde durch diese Linearbewegung in die Auslöselage der Abstand zum Sensor 45 so verringert, dass er ein Auslösesignal oder ein entsprechendes Messsignal, das das Erreichen der Auslöselage anzeigt, an die Steuereinheit S abgibt. Die Steuereinheit S steuert dann die Spannvorrichtung 17 an, die vorgespannte Kolbenstange 26 freizugeben, so dass das gewünschte nadellose Injizieren durchgeführt wird. Da der Sensor 45 gegenüber der Anschlagfläche 48 etwas zurückgesetzt ist, liegt selbst in der Auslöselage kein direkter Kontakt zwischen dem Sensor 45 und dem Magneten 32 vor.

Die Steuereinheit S kann das nadellose Injizieren basierend nur auf dem Messsignal des Sensors 45 ansteuern. Es ist jedoch vorteilhaft, wenn die Steuereinheit S dazu zusätzlich die Messsignale der beiden Kraftsensoren 82 und 83 sowie des Näherungssensors 85 auswertet. Durch die beiden Kraftsensoren 82 und 83 kann gut detektiert werden, dass das Huhn in der gedachten Art und Weise gegen die Anlagefläche 6 gedrückt wird. Sollte das Huhn schräg gegen die Anlagefläche 6 positioniert sein, würden die Werte der beiden Kraftsensoren 82 und 83 sich um einen vorbestimmten Wert unterscheiden, der anzeigen würde, dass kein nadelloses Injizieren sichergestellt werden kann. Des Weiteren kann das Messsignal des Näherungssensors 85 noch berücksichtigt werden, so dass eine größere Sicherheit dafür vorliegt, dass das Huhn in der vorbestimmten Art und Weise an der Anlagefläche 6 anliegt und gegen diese gedrückt wird.

Beim nadellosen Injizieren mittels des nadellosen Injektors 10 tritt konstruktionsbedingt im nadellosen Injektor 10 ein gewisser Rückschlag auf, der dazu führen könnte, das der gewünschte direkte Kontakt zwischen dem vorderen Abgabeende 9 und dem Brustmuskel (bzw. dem entsprechenden Hautbereich) gelöst wird, was zum einem verschlechterten nadellosen Injizieren führen kann. Daher ist die zweite Feder 47 an der Haltestange 33 im Halter 34 angeordnet, die aufgrund des Kontaktes zwischen dem Magneten 32 und der Anschlagfläche 48 eine zusätzliche Gegenkraft auf die Schlittenplatte 31 ausübt und diese zum vorderen Abgabeende 9 (und somit zur Anlagefläche 6) hin drückt. Die Kraft der zweiten Feder 47 wird somit in der Auslöselage zur Kraft der ersten Feder 43 zugeschaltet, so dass der Rückschlag des nadellosen Injektors 10 beim Injizieren kompensiert und damit der gewünschte direkte Kontakt zwischen dem vorderen Abgabeende 9 und dem Brustmuskel (bzw. dem entsprechenden Hautbereich) beibehalten wird.

An das nadellose Injizieren schließt sich wiederum das Vorspannen der Kolbenstange 26 und Halten des vorgespannten Zustands an. Jedoch kann ein erneutes nadelloses Injizieren erst dann wieder durchgeführt werden, wenn der Abstand zwischen dem Magneten 32 und dem Sensor 45 wieder dem Wert entspricht, wenn kein Druck auf das vordere Abgabeende 9 des nadellosen Injektors 10 ausgeübt wird (das entspricht dem Abnehmen des Tieres von der Anlageplatte 3). In dieser Art und Weise kann sichergestellt werden, dass dem jeweiligen Tier der Impfstoff oder das Medikament nur einmal nadellos injiziert wird.

Bei der beschriebenen Ausführungsform sind die zwei nadellose Injektoren 10, 12 vorgesehen, so dass zwei Impfstoffe und/oder Medikamente gleichzeitig nadellos injiziert werden können.

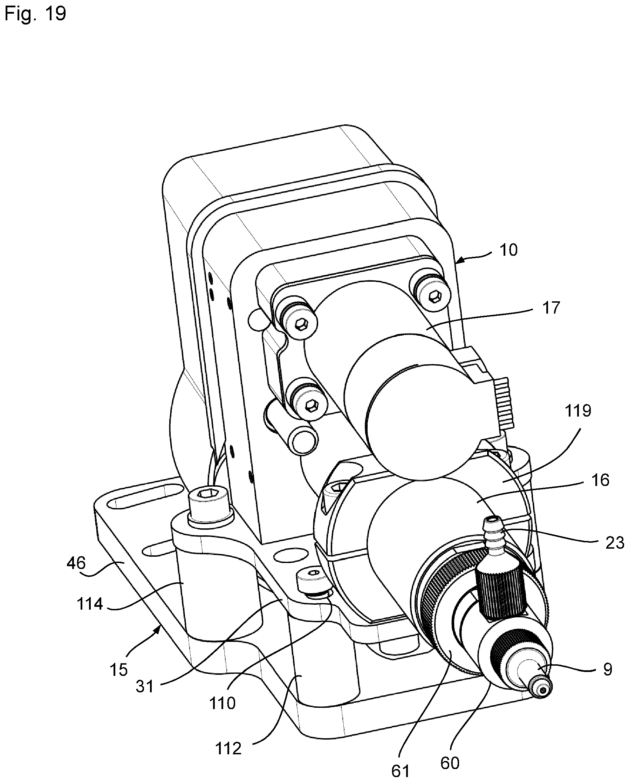

Eine weitere Ausführungsform der Vorrichtung 1 zum nadellosen Injizieren eines Fluids ist in Figuren 19 - 23 gezeigt, wobei sich im Vergleich zu der bisher beschriebenen Ausführungsform im Wesentlichen die Ausbildung der Linearführung 15 sowie die Anordnung von Kolben-Zylinder-Anordnung 16 zur Spannvorrichtung 17 unterscheidet. Die Details werden nachfolgen beschrieben.

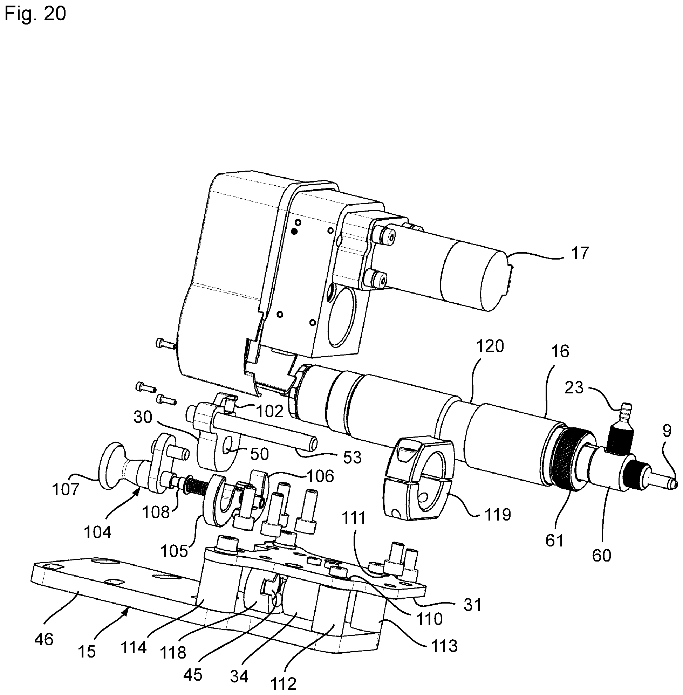

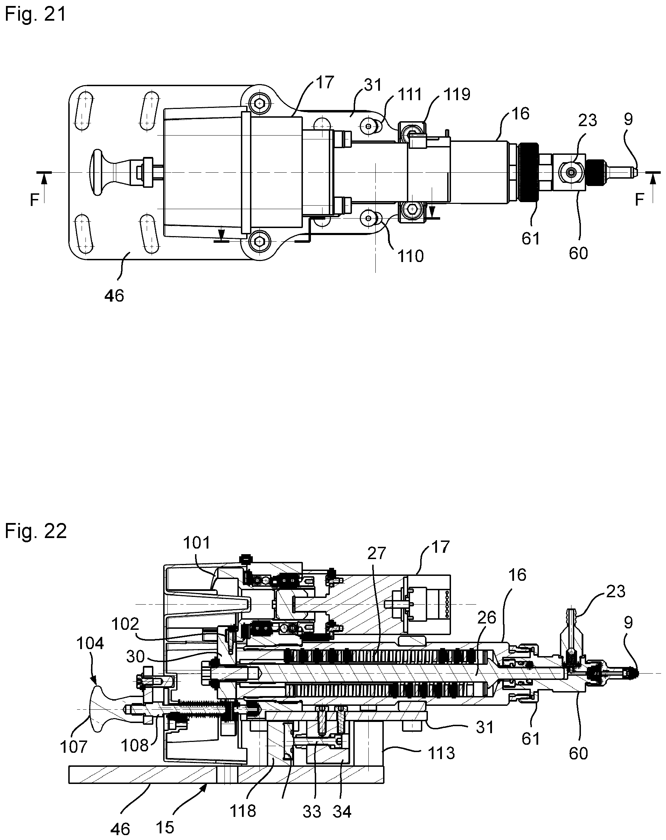

Wie insbesondere Figuren 19 - 21 zu entnehmen ist, ist die Spannvorrichtung 17 oberhalb der Kolben-Zylinder-Anordnung 16 (bezogen auf die Schlittenplatte 31) angeordnet. Dies führt zu einer etwas abgewandelten Ausbildung des Mitnehmers 30, wobei seine Walze 102 in Figuren 20 und 21 sichtbar und die Rampenbahn 101 der Spannvorrichtung 17 in Fig. 22 sichtbar ist.

Zusätzlich ist eine Dosiereinstelleinheit 104 mit einem ersten und einem zweiten einschwenkbaren Abstandshalter 105, 106 vorgesehen, die jeweils U-förmig ausgebildet sind (Fig. 20). Die Dosiereinstelleinheit 104 umfasst einen Drehknopf 107, der mit einem Ende einer Drehachse 108 verbunden ist, an deren anderem Ende die beiden Abstandshalter 105, 106 drehfest verbunden sind. Ein Drehen des Drehknopfs 107 führt somit zu einem Verschwenken der beiden Abstandshalter 105, 106. Bei der in Fig. 20 und 21 gezeigten neutralen Drehstellung des Drehknopfs 107 und somit der Drehstellung der beiden Abstandshalter 105, 106 ist keiner der beiden Abstandshalter 105, 106 zwischen dem Mitnehmer 30 und dem Anschlag des Mitnehmers 30 am hinteren Ende der Kolben-Zylinder-Anordnung 16. Somit liegt der maximale Hub des Kolbens 25 vor (die Kolbenstange steht in ihrer hinteren Endposition), der durch das obere Plateau der Rampenbahn 101 festgelegt ist.

Wenn die Rampenbahn 101 gedreht wird, um eine nadelloses Injizieren durchzuführen, läuft die Walze 102 über die Sprungkante, so dass keine Kraft zur Spannung der Feder 27 mehr vorliegt, wodurch die Kolbenstange 26 schlagartig zum vorderen Abgabeende 9 hin beschleunigt wird, bis der Mitnehmer 30 am hinteren Ende der Kolben-Zylinder-Anordnung 16 anliegt. Ein Drehen der Rampenbahn bis zum oberen Plateau führt dann zu einem nächsten Füllen des Zylinders.

In diesem Zustand kann dann durch Drehen des Drehknopfs 107 der erste oder zweite Abstandshalter 105, 106 zwischen dem Mitnehmer 30 und dem hinteren Ende der Kolben-Zylinder-Anordnung 16 positioniert werden. Die Ausdehnung des ersten oder zweiten Abstandshalters 105, 106 in Längsrichtung der Kolbenstange 26 entspricht dann der Verringerung des Hubs beim nadellosen Injizieren, da beim nadellosen Injizieren die Bewegung der Kolbenstange 26 bei Kontakt des Mitnehmers 30 mit dem ersten oder zweiten Abstandshalter 105, 106 gestoppt wird. Dies führt zu einer geringeren Fluidmenge (bzw. einem geringeren Fluidvolumen), die beim nadellosen Applizieren abgegeben wird. Da hier der erste Abstandshalter 105 eine andere Ausdehnung in Längsrichtung der Kolbenstange 26 aufweist als der zweite Abstandshalter 106, können mittels der Abstandshalter 105, 106 zwei unterschiedliche Fluidmengen eingestellt werden, die jeweils kleiner sind als die Fluidmenge bei maximalen Hub. Somit können mit der beschriebenen Vorrichtung 1 zum nadellosen Injizieren eines Fluids drei verschiedene Fluidmengen abgegeben werden.

Der erste Abstandshalter 105 kann in Umfangsrichtung zwei, drei oder mehr Abschnitte (nicht gezeigt) umfassen, die jeweils eine unterschiedliche Ausdehnung in Längsrichtung der Kolbenstange 26 aufweisen. Gleiches gilt für den zweiten Abstandshalter 106. Damit sind mehr als drei verschiedene Fluidmengen einstellbar. Auch kann nur der erste oder zweite Abstandshalter vorgesehen sein.

Die Dosiereinstelleinheit 104 kann so ausgebildet sein, dass sie per Hand betätigbar ist, wie dies in der beschrieben Ausführungsform der Fall ist. Der Benutzer muss lediglich den Drehknopf 107 drehen. Natürlich kann die Dosiereinstelleinheit 104 auch so ausgebildet sein, dass die Drehung der Drehachse motorgetrieben ist.

Die Linearführung 15 umfasst die Schlittenplatte 15 und die Grundplatte 46, wobei die Schlittenplatte 15 zwei Langlöcher 110, 111 aufweist, die die Bewegung der Schlittenplatte 15 in Längsrichtung bzw. in Verschieberichtung ermöglichen. Dazu ist die Schlittenplatte 15 über zwei starre Zylinder 112, 113 sowie zwei elastische Zylinder 114, 115 mit der Grundplatte 46 verbunden.

Die starren Zylinder 112, 113, die hier Metallzylinder sind, weisen jeweils einen zylinderförmigen Führungsstift 116, 117 auf, der in das entsprechende Langloch 110, 111 einsteht und dessen Außendurchmesser so gewählt ist, dass eine Relativbewegung zwischen Führungsstift 116, 117 und Langloch 110, 111 in Verschieberichtung möglich ist.

Die elastische Zylinder 114, 115 sind so elastisch, dass bei üblichen Kräften auf das vordere Ende 9 bei Andrücken eines Tieres an die Anlagefläche 6 der Anlageplatte 3 ein elastisches Verformen möglich ist und damit eine Bewegung der Schlittenplatte 31 relativ zur Grundplatte 46 in Verschieberichtung aufgrund der Führung durch die Langlöcher 110, 111 erfolgt.

Zur Detektion dieser Verschiebung der Schlittenplatte 31 ist an der Unterseite der Schlittenplatte 31 der Halter 34 mit der Haltestange 33 und dem Magneten 32 moniert. An der Grundplatte 46 ein Sensorhalter 118 montiert, in dem der Sensor 45 (hier z.B. der Hall-Sensor 45) angeordnet ist.

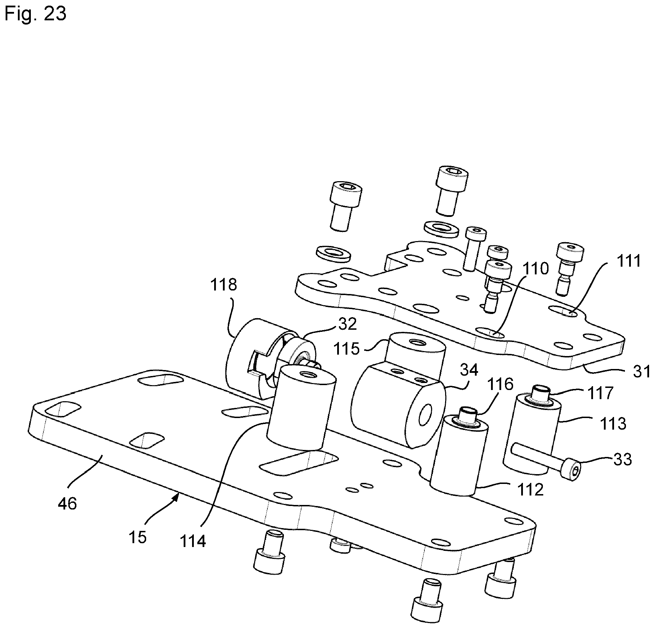

Die Zylinder 112-115 sind mit den in den Figuren (z.B. Figur 23) Schrauben mit der Schlittenplatte 31 und der Grundplatte 46 verschraubt. Auch die Befestigung des Halters 34 an der Schlittenplatte 31 und des Sensorhalters 118 an der Grundplatte 46 erfolgt durch Schrauben. Diese Schrauben sind in den Figuren 19 - 23 dargestellt, jedoch nicht mit Bezugszeichen versehen. Es ist natürlich auch jede andere Art der Befestigung bzw. des Verbindens möglich. Die Kolben-Zylinder-Anordnung 16 ist z.B. mittels einer mit der Schlittenplatte 31 verschraubten Manschette 119 (z.B. ein Klemm-Manschette), die in Eingriff mit einer Außennut 120 an der Kolben-Zylinder-Anordnung 16 steht, an der Schlittenplatte 31 befestigt.

Beim bestimmungsgemäß Gebrauch der Vorrichtung 1 zum nadellosen Injizieren eines Fluids Fluids steht der nadellose Injektor 10 aufgrund der Linearführung 15 federvorgespannt (bedingt durch die elastischen Zylinder 114, 115) in seiner vorderen Endlage (z.B. in Fig. 24 gezeigt), in der sich das vordere Abgabeende 9 durch die erste Durchgangsöffnung 7 hindurch erstreckt. Wird nun das Tier (hier ein Huhn mit seinem Brustbereich) gegen die geformte Anlagefläche 6 gedrückt, kommt dadurch das vordere Abgabeende 9 durch das Federkleid in Kontakt mit dem Brustmuskel (bzw. dem entsprechenden Hautbereich). Aufgrund der Linearführung 15 führt ein Druck auf das vordere Abgabeende 9 des nadellosen Injektors 10 zu einer Bewegung des nadellosen Injektors 10 mit der Schlittenplatte 31 in Richtung zum Sensorhalter 118 gegen die Federkraft der beiden elastischen Zylinder 114, 115. Ein Verschieben der Schlittenplatte 31 führt somit zu einer Bewegung des Magneten 32 in Verschieberichtung, wodurch der Abstand zwischen Magnet 32 und Sensor 45 geringer wird. Wenn der hintere Anschlag der Schlittenplatte 31 in Verschieberichtung erreicht ist (definiert durch die Ausdehnung des Langlochs 110, 111 in Verschieberichtung), liegt ein Abstand zwischen Magnet 32 und Sensor 45 vor, der zu einem Sensorsignal führt, dass als Anliegen des Tieres interpretiert wird. Diese Position der Schlittenplatte 31 kann auch als Auslöselage bezeichnet werden. Damit kann dann in der beschriebenen Art und Weise das nadellose Injizieren durchgeführt werden.

Die Auslöselage muss der hintere Anschlag der Schlittenplatte 31 sein. Es kann auch eine Position vor dem hinteren Anschlag als Auslöselage festgelegt werden. Es soll lediglich sichergestellt werden, dass der gewünschte Kontakt zwischen der Haut des Tieres (hier Brustmuskel des Huhns) und dem vorderen Abgabeende 9 vorliegt, um das gewünschte nadellose Injizieren durchführen zu können.

Anstatt der beschriebenen Messung mittel des Hall-Sensors 45 kann auch ein Kraftsensor vorgesehen sein, der dann die Kraft misst, mit der das entsprechende Ende 32 der Haltestange 33 gegen den Kraftsensor 45 gedrückt wird. In diesem Fall muss das Ende 32 keinen Magneten aufweisen.

Natürlich können auch bei dieser Ausführungsform die Messsignale der beiden Kraftsensoren 82, 83 sowie des Näherungssensors 85 in der beschriebenen Art und Weise ausgewertet werden, um das nadellose Injizieren durchzuführen.

Bei den bisher beschriebenen Ausführungsformen ist die Anlageplatte 3 so am Gehäuse 2 gelagert, dass sie nicht in Verschieberichtung verschiebbar ist. Es ist jedoch möglich, die Anlageplatte 3 in Verschieberichtung verschiebbar am Gehäuse 2 zu lagern, wie z.B. in Figuren 27 - 29 gezeigt ist.

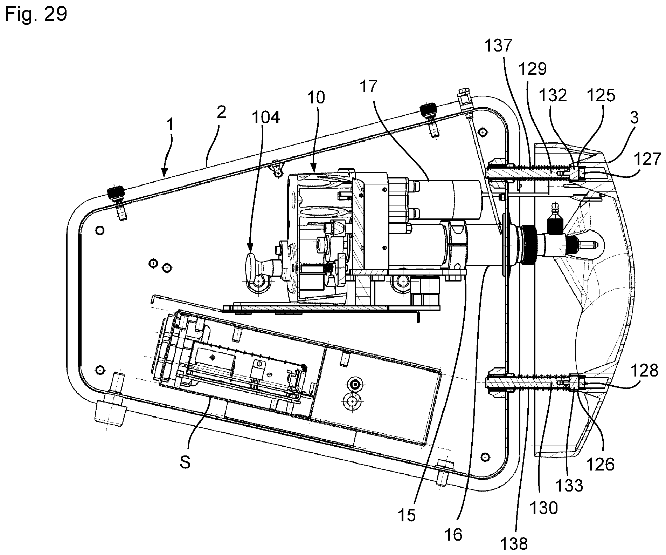

Die Anlageplatte 3 weist auf ihrer zum Gehäuse 2 weisenden Seite vier hohlzylinderförmige Aufnahmen (in der Schnittansicht von Fig. 29 sind zwei Aufnahmen 125, 126 sichtbar) auf, in den jeweils ein Magnet 127, 128 sitzt. Von der Vorderseite 5 des Gehäuses 2 stehen vier Führungsstangen 129, 130, 131, 132 vor, die in die vier hohlzylinderförmigen Aufnahmen 125, 126 einstehen. An den zur Anlageplatte 3 weisenden freien Enden Führungsstangen 129, 130, 131, 132 sind Magnete 133, 134, 135, 136 angeordnet, so dass die auf die Führungsstangen 129, 130, 131, 132 aufgeschobene Anlageplatte 3 magnetisch fixiert ist.

Die vier Führungsstangen 129, 130, 131, 132 sind jeweils verschiebbar zur Vorderseite 5 des Gehäuses 2 so gelagert, dass sie in das Gehäuse 2 eingeschoben werden können. Die vier Führungsstangen 129, 130, 131, 132 sind jedoch jeweils durch eine Feder 137, 138 in Richtung zur Anlageplatte 3 hin so vorgespannt, dass die Anlageplatte 3, wenn kein Tier gegen die Anlageplatte 3 gedrückt wird, in ihrer Grundposition steht, die in Fig. 29 gezeigt ist. In dieser Grundposition erstrecken sich die vorderen Enden 9, 11 der beiden nadellosen Injektoren 10, 12 nicht durch die beiden Durchgangsöffnungen 7, 8 in der geformten Anlagefläche 6 und stehen somit nicht gegenüber der geformten Anlagefläche 6 in einem Bereich angrenzend an die beiden Durchgangsöffnungen 7, 8 vor.

Wenn nun ein Tier gegen die Anlageplatte 3 gedrückt wird, wird dadurch die Anlageplatte 3 in Richtung zur Vorderseite 5 bewegt (die Führungsstangen 129, 130, 131, 132 tauchen in das Gehäuse 2 ein), bis die Anlageplatte 3 an der Vorderseite 5 anliegt. In dieser Injektionsposition der Anlageplatte 3 stehen die vorderen Enden 9, 11 der beiden nadellosen Injektoren 10, 12 gegenüber der geformten Anlagefläche 6 vor, so dass auch bereits ein Kontakt zwischen dem Brustmuskel und den vorderen Abgabeenden 9, 11 vorliegt, der die nadellosen Injektoren aufgrund der beschriebenen Linearführung 15 bis zur Auslöseposition bewegt hat. Somit wird das nadellose Injizieren durchgeführt, wobei sichergestellt ist, dass das Huhn richtig angelegt ist.

Die beschrieben Anlageplatte 3 ist bevorzugt so ausgebildet, dass sich alle Teile der Anlageplatte 3 in gleicher Weise bewegen, wenn ein Tier dagegen gedrückt wird. Insbesondere kann die Anlageplatte 3 einteilig ausgebildet sein.

Die Anlageplatte 3 kann beispielsweise auch zweiteilig ausgebildet sein, wobei ein innerer Teil, der mindestens einen Teil der geformten Anlagefläche 6 bildet, gegenüber einem äußeren Teil der Anlageplatte 3 in Verschieberichtung verschiebbar ist. Dies kann insbesondere so ausgebildet sein, wie dies in der DE 10 2015 122 069 A1 beschrieben ist. Es wird insbesondere auf Figuren 1 - 5 und der Beschreibung in den Absätzen Nr. 81 - 93 der DE 10 2015 122 069 A1 Bezug genommen.

## Patentansprüche

1. Vorrichtung zum nadellosen Injizieren eines Fluids in ein Tier, mit
einer Anlageeinrichtung (3), die eine geformte Anlagefläche (6) aufweist, die entsprechend einem Körperteil des Tieres geformt ist, dem die Injektion verabreicht werden soll,
einem ersten Injektor (10) zum nadellosen Injizieren, der ein vorderes Abgabeende (9) aufweist, das sich beim nadellosen Injizieren durch eine erste Durchgangsöffnung (7) in der geformten Anlagefläche (6) erstreckt und gegenüber der geformten Anlagefläche (6) in einem Bereich angrenzend an die erste Durchgangsöffnung (7) vorsteht,
einer ersten Messeinrichtung (32, 45), die mindestens ein erstes Messsignal abgibt, um einen Kontakt des Tieres mit dem vorderen Abgabeende (9) des ersten Injektors (10) zu detektieren, und
einer Steuereinheit (S), die ein nadelloses Injizieren mittels des ersten Injektors (10) basierend auf dem mindestens einen ersten Messsignal aktiviert.

2. Vorrichtung nach Anspruch 1, mit
einer ersten Linearführung (15), die den ersten Injektor (10) trägt, entlang einer ersten Verschieberichtung zwischen einer vorderen Endlage und einer Auslöselage bewegbar lagert und in der vorderen Endlage federvorgespannt hält wobei die erste Messeinrichtung (32, 45) bei Erreichen der Auslöselage durch den ersten Injektor (10) ein erstes Messsignal abgibt.

3. Vorrichtung nach Anspruch 1 oder 2, bei der
die Anlagefläche (6) zwischen einer Grundposition und einer Injektionsposition verschiebbar gelagert ist,
wobei sich das vordere Abgabeende (9) des ersten Injektors (10) in der Grundposition der Anlagefläche (6) nicht durch die erste Durchgangsöffnung (7) in der geformten Anlagefläche (6) erstreckt und sich das vordere Abgabeende (9) des ersten Injektors (10) in der Injektionsposition der Anlagefläche (6) durch die erste Durchgangsöffnung (7) in der geformten Anlagefläche (6) erstreckt und gegenüber der geformten Anlagefläche (6) in einem Bereich angrenzend an die erste Durchgangsöffnung (7) vorsteht.

4. Vorrichtung nach Anspruch 3, wobei
die Anlagefläche (6) so gelagert ist, dass die Anlagefläche (6) sich ohne dagegen gedrücktes Tier in der Grundposition befindet und dass die Anlagefläche (6) erst bei Überschreiten einer vorbestimmten Kraft durch Andrücken des Tiers in die Injektionsposition bewegbar ist.

5. Vorrichtung nach Anspruch 3 oder 4, bei der
die Anlagefläche (6) federvorgespannt in der Grundposition gehalten ist.

6. Vorrichtung nach mindestens Anspruch 2, bei der
die erste Messeinrichtung (32, 45) kontaktlos das Erreichen der Auslöselage detektiert.

7. Vorrichtung nach einem der obigen Ansprüche, bei der
das beim nadellosen Injizieren abgegebene Fluidvolumen einstellbar ist.

8. Vorrichtung nach einem der obigen Ansprüche, bei der
die erste Messeinrichtung (32, 45) eine auf den ersten Injektor (10) ausgeübte Kraft durch Andrücken des Tiers an die Anlagefläche (6) und damit gegen das vordere Abgabeende (9) des ersten Injektors (10) detektiert und erste Messsignale an die Steuereinheit (S) abgibt, die bei Überschreiten eines vorbestimmten Grenzwertes das nadellose Injizieren aktiviert.

9. Vorrichtung nach einem der obigen Ansprüche, bei der
die erste Messeinrichtung (32, 45) eine auf die Anlagefläche ausgeübte Kraft durch Andrücken des Tiers an die Anlagefläche (6) detektiert und erste Messsignale an die Steuereinheit (S) abgibt, die bei Überschreiten eines vorbestimmten Grenzwertes das nadellose Injizieren auslöst.

10. Vorrichtung nach einem der obigen Ansprüche, bei der
die erste Messeinrichtung (32, 45) einen Näherungssensor (85) aufweist, der das Vorhandensein des Tiers an der Anlagefläche (6) detektiert und ein erstes Messsignal an die Steuereinheit (S) abgibt, die basierend auf dem ersten Messsignal des Näherungssensors (85) das nadellose Injizieren auslöst.

11. Vorrichtung nach einem der obigen Ansprüche, bei der
der erste Injektor (10) als Selbstfüllerspritze ausgebildet ist.

12. Vorrichtung nach einem der obigen Ansprüche, mit
einem zweiten Injektor (12) zum nadellosen Injizieren, der ein vorderes Abgabeende (11) aufweist, das sich beim nadellosen Injizieren durch eine zweite Durchgangsöffnung (7) in der geformten Anlagefläche (6) erstreckt und gegenüber der geformten Anlagefläche (6) in einem Bereich angrenzend an die zweite Durchgangsöffnung (7) vorsteht, und
einer zweiten Messeinrichtung (32, 45), die mindestens ein zweites Messsignal abgibt, um einen Kontakt des Tieres mit dem vorderen Abgabeende (11) des zweiten Injektors (12) zu detektieren,
wobei die Steuereinheit (S) ein nadelloses Injizieren mittels des zweiten Injektors (10) basierend auf dem mindestens einen zweiten Messsignal aktiviert.
